# EUROPEAN PATENT SPECIFICATION

(11) **EP 2 386 624 B1**
(45) Date of publication and mention of the grant of the patent: **23.07.2014**
(21) Application number: 10832504.4
(22) Date of filing: 03.11.2010
(51) Int. Cl.: C12N 1/18, C12N 1/20, A61K 36/064, A61K 35/74, A61P 3/10, A61K 36/06

(54) **COMPLEX MICROBIAL PREPARATION FOR TREATING DIABETES AND PREPARATIVE METHOD AND USE THEREOF**
KOMPLEXES MIKROBIELLES PRÄPARAT ZUR BEHANDLUNG VON DIABETES SOWIE VERFAHREN ZU SEINER HERSTELLUNG UND VERWENDUNG
PRÉPARATION MICROBIENNE COMPLEXE POUR LE TRAITEMENT DU DIABÈTE ET SON PROCÉDÉ DE PRÉPARATION ET SON UTILISATION

(30) Priority: 25.11.2009 CN 200910238778
(43) Date of publication of application: 16.11.2011
(73) Proprietor: Wang, Liping, Beijing 100089 (CN)
(72) Inventor: Wang, Liping, Beijing 100089 (CN)
(74) Representative: Novagraaf Technologies
(86) International application number: PCT/CN2010/001769
(87) International publication number: WO 2011/063597

(56) References cited:
- EP-A1- 2 030 623
- WO-A1-2005/032568
- WO-A1-2009/138448
- CN-A- 1 357 360
- CN-A- 1 875 738
- CN-A- 101 078 003
- CN-A- 101 703 528
- CHEN MIN: 'Microbial mixed culture fermentation and its applications' JOURNAL OF HANGZHOU NORMAL COLLEGE no. 6, 30 November 1991, pages 88 - 94

## Description

### Technical Field

The present invention belongs to the field of biotechnology, in particular, relates to a complex microbial preparation and preparative method thereof and its use in producing a medicament for treatment of diabetes.

### Background Art

Diabetes is a common, frequently-occurring and lifelong disease, the cause of the disease is that the body can not normally release or use insulin and lead to an improper increase of the level of glucose (a kind of monosaccharide) in blood. The investigation of the World Health Organization (WHO) shows that, at present, the population of patients with diabetes in the world is up to about 160 millions, and there are over 60 millions diabetes patients in China, which accounts for one third of the patients with diabetes in the world. According to statistics, among people with diseases caused by diabetes, people with blindness are 10-23 times, people with gangrene and amputation are 20 times, people with kidney failure are 17 times, people with coronary disease and apoplexy are 2-3 times, and people with sudden death are 3-8 times more than those in ordinary people. For this reason, the WHO appealed that it is of vital importance to treat diabetes as early as possible for reducing the risk of complications.

Drug therapy for diabetes includes insulin therapy, chemical drug therapy by oral hypoglyceimic agents and traditional Chinese medicine therapy, and current biotechnology therapy. Chinese Patent No. 1270813 "A drug for treating diabetes" discloses that, a drug for treating diabetes is prepared with extract of metabolic products from single microbial strains. Chinese Patent No.00804999 "A medicament for treatment of diabetes" relates to physiologically acceptable enzyme mixtures originated from microbe or animal. In Chinese Patent No.01130450, the "Biological hypoglycemic agents" consists of three microorganisms i.e. *Actinomycetes, Lactobacillus* and Yeasts, and is prepared through conventional activation, enlarged culture, fermentation, filtering, dreg elimination, disinfection, freeze drying and other steps.

For the treatment of diabetes, the chemical drug is expensive, and it can bring significant side effects and only cure the symptom, not the disease; the traditional Chinese medicine is slow to take effect and needs long time for treating; while the biotechnology therapy can overcome the drawbacks of the above two medicines. In Chinese Patent No.99105621, "Biological hypoglycemic agent" is a single microbial strain with a sole therapeutic effect. The medicament in Chinese Patent No.00804999 is an enzymatic preparation as an aid in the treatment of diabetes. In Chinese Patent No.01130450, although the hypoglycemic biopreparation consists of three microorganisms i.e. *Actinomycetes, Lactobacillus* and Yeasts, however, it is only a simple mixture, the blending ratio of the three microbes was not defined; there are not certain amount of clinical verifications, and its clinical effect needs to be evaluated.

### Contents of the Invention

The object of the invention is to provide a complex microbial preparation for use in the treatment of diabetes. The complex microbial preparation according to the invention mainly comprises of a plurality of probiotics components including *Photosynthetic bacterium, Bacillus,* Yeasts, *Lactobacillus* and *Actinomycetes.*

Wherein the above mentioned components are respectively 5-15% by volume of *Photosynthetic bacterium,* 10-20% by volume of *Bacillus,* 20-35% by volume of Yeasts, 30-45% by volume of *Lactobacillus* and 3-10% by volume of *Actinomycetes*; preferably 5-10% by volume of *Photosynthetic bacterium,* 10-14% by volume of *Bacillus,* 30-35% by volume of Yeasts, 41-45% by volume of *Lactobacillus* and 3-8% by volume of *Actinomycetes* by volume; more preferably 10% by volume of *Photosynthetic bacterium,* 10% by volume of *Bacillus,* 30% by volume of Yeasts, 45% by volume of *Lactobacillus* and 5% by volume of *Actinomycetes*;

The *Photosynthetic bacterium* is preferably *Rhodopseudomonas palustris,* the *Bacillus* is preferably *Bacillus subtilis,* the Yeasts is preferably *Saccharomyces cerevisiae,* the *Lactobacillus* is preferably *Lactobacillus acidophilus* and the *Actinomycetes* is preferably *Streptomyces jingyangensis*.

Another object of the invention is to provide a preparative method of the complex microbial preparation. The preparative method comprises the steps of first and second level complex fermentations of *Photosynthetic bacterium, Bacillus,* Yeasts, *Lactobacillus* and *Actinomycetes,* respectively.

The first level complex fermentation comprises steps of:
(1) separately inoculating mother seed of the *Photosynthetic bacterium, Bacillus,* Yeasts, *Lactobacillus* and *Actinomycetes* on first level culture media and conducting a first level cultivation to obtain first level bacteria;
(2) separately inoculating the obtained first level bacteria of the *Photosynthetic bacterium, Bacillus,* Yeasts, *Lactobacillus* and *Actinomycetes* on second level culture media and conducting a second level cultivation to obtain fermenting bacterial liquids;
(3) uniformly mixing the five obtained fermenting bacterial liquids in a specific ratio to obtain first level compound bacteria.

For the first level cultivation in step (1), the *Photosynthetic bacterium* are cultured with 0.5-1% of inoculation amount, preferably 0.5%, with a pH of 6-10 and a light intensity of 3,000-4,000 Lux, at 28-36°C for 7-10 days; the *Bacillus* are cultured with 0.5-1% of inoculation amount, preferably 0.5%, with a pH of 6-8, at 28-34°C for 2-3 days; the Yeasts are cultured with 0.5-1% of inoculation amount, preferably 0.5%, with a pH of 4-7, at 25-30°C for 1-2 days; the *Lactobacillus* are cultured with 0.5-1% of inoculation amount, preferably 0.5%, with a pH of 6-8, at 28-34°C for 2-5 days; and the *Actinomycetes* are cultured with 0.5-1% of inoculation amount, preferably 0.5%, with a pH of 6-8, at 27-32°C for 5-7 days;

For the second level cultivation in step (2), the *Photosynthetic bacterium* are preferably cultured by intermittent aerobic cultivation with 5-15% of inoculation amount, preferably 10%, with a pH of 6-10 and a light intensity of 3,000-4,000 Lux, at 28-36°C for 7-10 days; the *Bacillus* are preferably cultured by aerobic cultivation with 5-15% of inoculation amount, preferably 10%, with a pH of 6-8, at 28-32°C for 2-5 days; the Yeasts are preferably cultured by aerobic cultivation with 5-15% of inoculation amount, preferably 10%, with a pH of 4-7, at 25-30°C for 1-2 days; the *Lactobacillus* are preferably cultured by anaerobic cultivation with 5-15% of inoculation amount, preferably 10%, with a pH of 6-8, at 28-34°C for 2-5 days; and the *Actinomycetes* are preferably cultured by aerobic cultivation with 5-15% of inoculation amount, preferably 10%, with a pH of 6-8, at 27-32°C for 5-7 days.

The second level complex fermentation comprises steps of:
adding sterile water to the above first level compound bacteria to dilute it 2-4 times, then adding 10-20% of *Lactobacillus plantarum,* 10-20% of *Lactobacillus acidophilus,* 10-20% of *Saccharomyces cerevisiae* and 5-15% of brown sugar thereto, mixing uniformly in a fluid reservior, fermenting under sterile and closed condition, culturing with a pH of 5-8 at 25-35°C for 2-3 days to obtain second level compound bacteria.

The second level compound bacterial suspension is sampled and detected, results show: it has liquid appearance, uniform color and special odor produced by microorganism fermentation; pH value is directly measured with acidity meter and normal pH value is ranging from 5 to 8; the viable bacteria count and undesired bacteria count are measured by plate counting, a qualified product has the total viable bacteria count of more than 5 × 10⁸cfu/g and the aerobic bacteria count of 5% or less.

The object of the invention is to provide use of the complex microbial preparation in producing a medicament for treatment of diabetes. The complex microbial preparation according to the invention was administered to patients with diabetes three times a day with 20 ml each time. 15 days was taken as a course of treatment, and the patients recovered to normal blood glucose level after 3 courses of treatment with an efficiency of over 80%.

The key points of the invention are as follows:
1. Among the components of the complex microbial preparation according to the invention, *Photosynthetic bacterium* and *Bacillus* have the function of maintaining micro-ecological balance; Yeasts contains a plurality of proteins, vitamin B, nucleic acids and mineral substances capable of regulating metabolic functions of human body; *Lactobacillus* exhibits many physiological functions in animal body, e.g. reducing the level of serum cholesterol, controlling endotoxin, inhibiting the growth of intestinal spoilage bacteria, and improving immune function of organisms; *Actinomycetes* can produce antibiotic substances;
2. A plurality of probiotics including *Photosynthetic bacterium, Bacillus,* Yeasts, *Lactobacillus* and *Actinomycetes* in a specific ratio, two level complex fermentations are conducted to obtain a complex microbial preparation according to the invention ;
3. Utilizing the synergetic function of a plurality of beneficial bio-regulatory factors, amino acids, antibiotic substances, various complex microbial active proteins and active enzymes which are produced after fermentation, the complex microbial preparation according to the invention is used to produce medicaments for treating diabetes. The complex microbial preparation according to the invention can rapidly regulate metabolic function of human body, repair and recombine pancreatic islet cell mass, transform the redundant glucose in the blood, reduce the burden of impaired pancreatic β cells, gradually recover the function of the impaired pancreas islets, improve microcirculation perfusion , maintain steady state of the inter environment, keep the balance of organism, correct metabolic disorder and unbalance, fundamentally eliminate diabetes and the complications, improve life quality of diabetic patients, get rid of the clinical disadvantages of taking western medicine and strictly control the diet diabetes in a long term for the diabetic patients. The complex microbial preparation according to the invention is cost effective and rapid to take effect with a good taste, while applying the preparation, there is no need to control diet and both the symptoms and root cause can be treated.

The main efficacy of the complex microbial preparation according to the invention is as follows:
1. Repairing and recombining pancreatic islet cell mass, transforming the redundant glucose in the blood, reducing the burden of impaired pancreatic β cells, activating the repair factors, recovering the function of the impaired pancreas islets, inhibiting the occurrence of complications, improving self-immunity, enhancing circulation of micro-ecosystem, finally completely recovering the function of the pancreas islets and reaching a result of recovering fully and addressing both the symptoms and root cause.
2. Overall adjusting the body function of the patients by regulating metabolism to comprehensively control the blood glucose, restoring the ability of self-regulating the blood glucose of human body by activating self-function of the patients to adjust the blood glucose to a normal level.
3. Improving microcirculation perfusion to enhance oxygen and blood supply of cells ; improving blood perfusion to increase blood flow of tissues and treat and prevent diabetic micro-vascular complications and prevent fatal harm from diabetes.
4. Elevating the velocity of nerve transmission and increasing acral temperature, improving acral pruritus and prickling of diabetes, as well as preventing the occurrence of gangrene and amputation.
5. Improving sleep of the patients with neurological complication, correcting obstinate insomnia and the state of fatigue, lassitude etc.
6. Improving the diseases including diarrhea and constipation which are caused by functional disorders of the digestive system of diabetes, and hypertension caused by diabetes.
7. Maintaining steady state of the inter environment in human body, keeping balance of the organism, and correcting metabolic disorder and unbalance.

### Brief Description of the Drawings

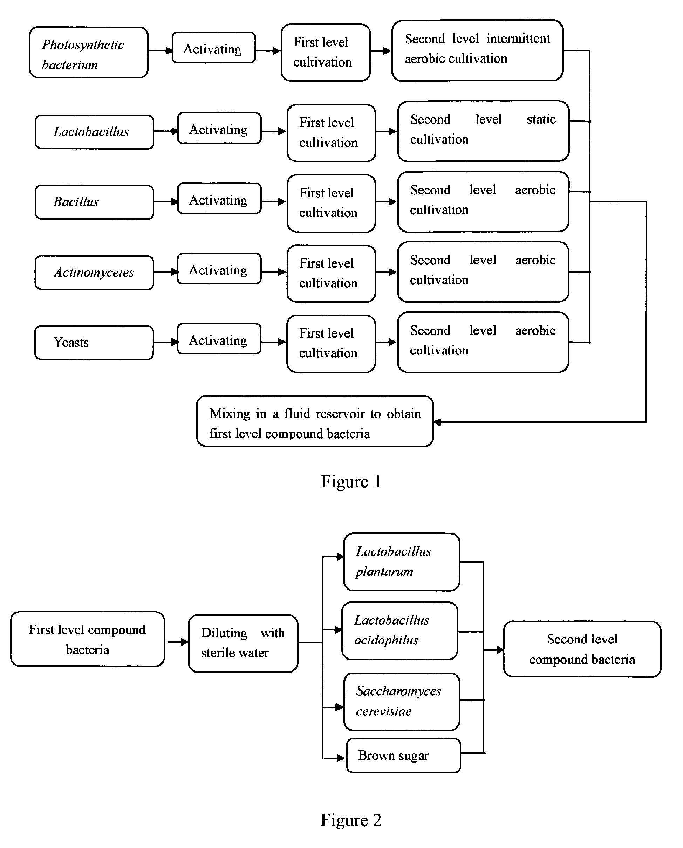

Figure 1 is a How diagram of first level complex fermentation;
Figure 2 is a flow diagram of second level complex fermentation.

### Specific Mode for Carrying Out Embodiments

Examples are described below to further explain the contents of the invention. The scope of the invention should not be limited by the examples.

### Medium for Various Bacteria

First level culture media for *Rhodopseudomonas palustris*: 0.2g of MgSO₄·7H₂O, 1.0g of (NH₄)₂SO₄, 5.0g of NaHCO₃, 0.5g of K₂HPO₄, 0.2g of NaCl and 1.5g of peptone were dissolved in 400ml of distilled water, the pH was adjust to 7.1 with H₃PO₄ and Na₂CO₃, then distilled water was added to adjust the volume to 1000ml, andsterilized at 115°C for 10 minutes; second level culture media: to 3g of Na₂CO₃, 0.1g of NaCl, 0.3g of (NH₄)₂SO₄, 0.2g of MgSO₄, 0.5g of KH₂PO₃, 0.3g of K₂HPO₃, 7ml of Ferric ammonium citrate and 1.2g of peptone was added distilled water to make a total volume of 1000ml, and the pH was adjusted to 7.2 with H₃PO₄ and Na₂CO₃.

First level culture media for *Bacillus subtilis*: to 0.3% of Beef extract, 1.0% of peptone and 0.5% of NaCl was added distilled water to make a total volume of 1000ml, pH 7.0; second level culture media: to 1.0% of Beef extract, 1.0% of peptone and 0.5% of NaCl was added distilled water to make a total volume of 1000ml, pH 7.0.

First level culture media for *Saccharomyces cerevisiae*: to 800ml of potato juice, 20g of glucose, 20g of agar was added distilled water to make a total volume of 1000ml, pH 6.0; second level culture media: to 1% of yeast extract, 2% of peptone, 2% of glucose, 0.06% of MgSO₄ and 0.25% of K₂HPO₄ was added water to make a total of 100%, pH 6.0.

First level culture media for *Lactobacillus acidophilus*: to 1g of KNO₃, 10g soluble starch, 0.5g of K₂HPO₄, 0.5g of MgSO₄, 0.5g of NaCl, 0.01g of FeSO₄ and 20g of agar was added distilled water to make a total volume of 1000ml, pH 7.2; second level culture media: to 0.8g of KNO₃, 8g of soluble starch, 5g of glucose, 5g of yeast juice, 0.3g of K₂HPO₄, 0.2g of MgSO₄, 0.4g of NaCl and 0.05g of FeSO₄ was added distilled water to make a total volume of 1000ml, pH7.2.

First level culture media for *Streptomyces jingyangensis*: to 1g of KNO₃, 20g of soluble starch, 0.5g of K₂HPO₄, 0.5g of MgSO₄, 0.5g of NaCl, 0.01g of FeSO₄ and 20g of agar was added distilled water to make a total volume of 1000ml, pH 7.2; second level culture media: to 0.8g of KNO₃, 8g of soluble starch, 5g of glucose, 5g of yeast juice, 0.3g of K₂HPO₄, 0.2g of MgSO₄, 0.4g of NaCl, 0.05g of FeSO₄ was added distilled water to make a total volume of 1000ml, pH7.2.

### Example 1 Preparative method of the complex microbial preparation

First level complex fermentation:
(1) 300ml of eggplant bottle strains of *Rhodopseudomonas palustris* was inoculated in 60L of the first level culture media for *Rhodopseudomonas palustris,* cultured at 28°C with a pH of 6 and light intensity of 3000 Lux for 10 days to obtain first level bacteria of *Rhodopseudomonas palustris;*
   300ml of eggplant bottle strains of *Bacillus subtilis* was inoculated in 60L of the first level culture media for *Bacillus subtilis,* cultured at 28°C with a pH of 6 for 3 days to obtain first level bacteria of *Bacillus subtilis;*
   300ml of eggplant bottle strains of *Saccharomyces cerevisiae* was inoculated in 60L of the first level culture media for *Saccharomyces cerevisiae,* cultured at 25°C with a pH of 4 for 2 days to obtain first level bacteria of *Saccharomyces cerevisiae;*
   300ml of eggplant bottle strains of *Lactobacillus acidophilus* was inoculated in 60L of the first level culture media for *Lactobacillus acidophilus,* cultured at 28°C with a pH of 6 for 5 days to obtain first level bacteria of *Lactobacillus acidophilus;*
   300ml of eggplant bottle strains of *Streptomyces jingyangensis* was inoculated in 60L of the first level culture media for *Streptomyces jingyangensis,* cultured at 27°C with a pH of 6 for 7 days to obtain first level bacteria of *Streptomyces jingyangensis;*
(2) 60L of the first level bacteria *of Rhodopseudomonas palustris* was inoculated in 600L of second level culture media for *Rhodopseudomonas palustris,* cultured by intermittent aerobic cultivation at 28°C with a pH of 6 and light intensity of 3000 Lux for 10 days to obtain fermenting bacterial suspension of *Rhodopseudomonas palustris;* with determination, viable count of the bacteria in the fermenting bacterial suspension is 5×10⁸-10×10⁸.
   60L of the first level bacteria of *Bacillus subtilis* was inoculated in 600L of second level culture media for *Bacillus subtilis,* cultured by aerobic cultivation at 28°C with a pH of 6 for 3 days to obtain fermenting bacterial suspension of *Bacillus subtilis;* with determination, viable count of the bacteria in the fermenting bacterial suspension is 30×10⁸-50×10⁸.
   60L of the first level bacteria of *Saccharomyces cerevisiae* was inoculated in 600L of second level culture media for *Saccharomyces cerevisiae,* cultured by aerobic cultivation at 25°C with a pH of 4 for 2 days to obtain fermenting bacterial suspension of *Saccharomyces cerevisiae*; with determination, viable count of the bacteria in the fermenting bacterial suspension is 5×10⁸-10×10⁸.
   60L of the first level bacteria of *Lactobacillus acidophilus* was inoculated in 600L of second level culture media for *Lactobacillus acidophilus*, cultured by static anaerobic cultivation at 28°C with a pH of 6 for 5 days to obtain fermenting bacterial suspension of *Lactobacillus acidophilus*; with determination, viable count of the bacteria in the fermenting bacterial suspension is 20×10⁸ -30×10⁸.
   60L of the first level bacteria of *Streptomyces jingyangensis* was inoculated in 600L of second level culture media for *Streptomyces jingyangensis*, cultured by aerobic cultivation at 27°C with a pH of 6 for 7 days to obtain fermenting bacterial suspension of *Streptomyces jingyangensis*; with determination, viable count of the bacteria in the fermenting bacterial suspension is 30×10⁸-50×10⁸.
(3) Various components of fermenting bacterial suspension were uniformly mixed in a 1000 L fluid reservoir with a volume ratio of: 10% of *Rhodopseudomonas palustris,* 10% of *Bacillus subtilis,* 30% of *Saccharomyces cerevisiae*, 45% of *Lactobacillus acidophilus* and 5% of *Streptomyces jingyangensis*, obtaining 1000L of first level compound bacteria.

Second level complex fermentation:
2450 L of sterile water was added to 1000 L of the first level compound bacteria to dilute it, then 150 L of *Lactobacillus plantarum*, 150 L of *Lactobacillus acidophilus*, 150 L of *Saccharomyces cerevisiae* and 100 L of brown sugar were added to mix uniformly in a fluid reservoir, fermented under sterile and closed condition, cultured at 30°C with a pH of 7 for 3 days to obtain 4000 L of second level compound bacteria.

With determination, total count of the viable bacteria in the compound bacteria is 20×10⁸-30×10⁸. Wherein, there were 8% of *Rhodopseudomonas palustris*, 12% of *Bacillus subtilis,* 32% of *Saccharomyces cerevisiae*, 44% of *Lactobacillus acidophilus* and 4% of *Streptomyces jingyangensis.*

### Example 2 Preparative method of the complex microbial preparation

First level complex fermentation:
(1) 500ml of eggplant bottle strains of *Rhodopseudomonas palustris* was inoculated in 60L of the first level culture media for *Rhodopseudomonas palustris*, cultured at 32°C with a pH of 8 and light intensity of 3500 Lux for 8 days to obtain first level bacteria of *Rhodopseudomonas palustris*;
   500ml of eggplant bottle strains of *Bacillus subtilis* was inoculated in 60L of the first level culture media for *Bacillus subtilis,* cultured at 32°C with a pH of 7 for 2 days to obtain first level bacteria of *Bacillus subtilis;*
   500ml of eggplant bottle strains of *Saccharomyces cerevisiae* was inoculated in 60L of the first level culture media for *Saccharomyces cerevisiae*, cultured at 28°C with a pH of 5 for 1 days to obtain first level bacteria of *Saccharomyces cerevisiae*;
   500ml of eggplant bottle strains of *Lactobacillus acidophilus* was inoculated in 60L of the first level culture media for *Lactobacillus acidophilus*, cultured at 32°C with a pH of 7 for 3 days to obtain first level bacteria *of Lactobacillus acidophilus;*
   500ml of eggplant bottle strains of *Streptomyces jingyangensis* was inoculated in 60L of the first level culture media for *Streptomyces jingyangensis*, cultured at 30°C with a pH of 7 for 6 days to obtain first level bacteria of *Streptomyces jingyangensis*;
(2) 60L of the first level bacteria of *Rhodopseudomonas palustris* was inoculated in 600L of second level culture media for *Rhodopseudomonas palustris*, cultured by intermittent aerobic cultivation at 32°C with a pH of 8 and light intensity of 3500Lux for 8 days to obtain fermenting bacterial suspension of *Rhodopseudomonas palustris*; with determination, viable count of the bacteria in the fermenting bacterial suspension is 5×10⁸-10×10⁸.
   60L of the first level bacteria of *Bacillus subtilis* was inoculated in 600L of second level culture media for *Bacillus subtilis,* cultured by aerobic cultivation at 32°C with a pH of 7 for 2 days to obtain fermenting bacterial suspension of *Bacillus subtilis;* with determination, viable count of the bacteria in the fermenting bacterial suspension is 30×10⁸-50×10⁸.
   60L of the first level bacteria of *Saccharomyces cerevisiae* was inoculated in 600L of second level culture media for *Saccharomyces cerevisiae*, cultured by aerobic cultivation at 28°C with a pH of 5 for 1 days to obtain fermenting bacterial suspension of *Saccharomyces cerevisiae*; with determination, viable count of the bacteria in the fermenting bacterial suspension is 5×10⁸-10×10⁸.
   60L of the first level bacteria of *Lactobacillus acidophilus* was inoculated in 600L of second level culture media for *Lactobacillus acidophilus*, cultured by static anaerobic cultivation at 32°C with a pH of 7 for 3 days to obtain fermenting bacterial suspension of *Lactobacillus acidophilus*; with determination, viable count of the bacteria in the fermenting bacterial suspension is 20×10⁸-30×10⁸.
   60L of the first level bacteria of *Streptomyces jingyangensis* was inoculated in 600L of second level culture media for *Streptomyces jingyangensis*, cultured by aerobic cultivation at 30°C with a pH of 7 for 6 days to obtain fermenting bacterial suspension of *Streptomyces jingyangensis*; with determination, viable count of the bacteria in the fermenting bacterial suspension is 30×10⁸-50×10⁸.
(3) Various components of comfermenting bacterial suspension were uniformly mixed in a 1000L fluid reservoir with a volume ratio of: 5% of *Rhodopseudomonas palustris*, 14% of *Bacillus subtilis,* 32% of *Saccharomyces cerevisiae*, 41% of *Lactobacillus acidophilus* and 8% of *Streptomyces jingyangensis*, obtaining 1000L of first level compound bacteria.

Second level complex fermentation:
2150 L of sterile water was added to 1000L of the first level compound bacteria to dilute it, then 100L of *Lactobacillus plantarum*, 100L of *Lactobacillus acidophilus*, 100L of *Saccharomyces cerevisiae* and 50L of brown sugar were added to mix uniformly in a fluid reservoir, fermented under sterile and closed condition, cultured at 25°C with a pH of 5 for 2 days to obtain 3500L of second level compound bacteria.

With determination, total count of the viable bacteria in the compound bacteria is 20×10⁸-30×10⁸. Wherein, there were 6% of *Rhodopseudomonas palustris*, 15% of *Bacillus subtilis,* 33% of *Saccharomyces cerevisiae*, 42% of *Lactobacillus acidophilus* and 4% of *Streptomyces jingyangensis.*

### Example 3 Preparative method of the complex microbial preparation

First level complex fermentation:
(1) 600ml of eggplant bottle strains of *Rhodopseudomonas palustris* was inoculated in 60L of the first level culture media for *Rhodopseudomonas palustris*, cultured at 36°C with a pH of 10 and light intensity of 4000Lux for 7 days to obtain first level bacteria of *Rhodopseudomonas palustris*;
   600ml of eggplant bottle strains of *Bacillus subtilis* was inoculated in 60L of the first level culture media for *Bacillus subtilis,* cultured at 34°C with a pH of 8 for 2 days to obtain first level bacteria of *Bacillus subtilis;*
   600ml of eggplant bottle strains of *Saccharomyces cerevisiae* was inoculated in 60L of the first level culture media for *Saccharomyces cerevisiae*, cultured at 30°C with a pH of 7 for 1 days to obtain first level bacteria of *Saccharomyces cerevisiae*;
   600ml of eggplant bottle strains of *Lactobacillus acidophilus* was inoculated in 60L of the first level culture media for *Lactobacillus acidophilus*, cultured at 34°C with a pH of 8 for 2 days to obtain first level bacteria of *Lactobacillus acidophilus;*
   600ml of eggplant bottle strains of *Streptomyces jingyangensis* was inoculated in 60L of the first level culture media for *Streptomyces jingyangensis*, cultured at 32°C with a pH of 8 for 5 days to obtain first level bacteria of *Streptomyces jingyangensis*;
(2) 60L of the first level bacteria of *Rhodopseudomonas palustris* was inoculated in 600L of second level culture media for *Rhodopseudomonas palustris*, cultured by intermittent aerobic cultivation at 36°C with a pH of 10 and light intensity of 4000Lux for 7 days to obtain fermenting bacterial suspension of *Rhodopseudomonas palustris*; with determination, viable count of the bacteria in the fermenting bacterial suspension is 5×10⁸-10×10⁸.
   60L of the first level bacteria of *Bacillus subtilis* was inoculated in 600L of second level culture media for *Bacillus subtilis,* cultured by aerobic cultivation at 34°C with a pH of 8 for 2 days to obtain fermenting bacterial suspension of *Bacillus subtilis;* with determination, viable count of the bacteria in the fermenting bacterial suspension is 30×10⁸-50×10⁸.
   60L of the first level bacteria of *Saccharomyces cerevisiae* was inoculated in 600L of second level culture media for *Saccharomyces cerevisiae*, cultured by aerobic cultivation at 30°C with a pH of 7 for 1 days to obtain fermenting bacterial suspension of *Saccharomyces cerevisiae*; with determination, viable count of the bacteria in the fermenting bacterial suspension is 5×10⁸-10×10⁸.
   60L of the first level bacteria of *Lactobacillus acidophilus* was inoculated in 600L of second level culture media for *Lactobacillus acidophilus*, cultured by static anaerobic cultivation at 34°C with a pH of 8 for 2 days to obtain fermenting bacterial suspension of *Lactobacillus acidophilus*; with determination, viable count of the bacteria in the fermenting bacterial suspension is 20×10⁸-30×10⁸.
   60L of the first level bacteria of *Streptomyces jingyangensis* was inoculated in 600L of second level culture media for *Streptomyces jingyangensis*, cultured by aerobic cultivation at 32°C with a pH of 8 for 5 days to obtain fermenting bacterial suspension of *Streptomyces jingyangensis*; with determination, viable count of the bacteria in the fermenting bacterial suspension is 30×10⁸-50×10⁸.
(3) Various components of comfermenting bacterial suspension were uniformly mixed in a 1000L fluid reservoir with a volume ratio of: 8% of *Rhodopseudomonas palustris,* 12% of *Bacillus subtilis,* 35% of *Saccharomyces cerevisiae*, 42% of *Lactobacillus acidophilus* and 3% of *Streptomyces jingyangensis*, obtaining 1000L of first level compound bacteria.

Second level complex fermentation:
3250 L of sterile water was added to 1000L of the first level compound bacteria to dilute it, then 200L of *Lactobacillus plantarum*, 200L of *Lactobacillus acidophilus*, 200L of *Saccharomyces cerevisiae* and 150L of brown sugar were added to mix uniformly in a fluid reservoir, fermented under sterile and closed condition, cultured at 35°C with a pH of 8 for 3 days to obtain 5000L of second level compound bacteria.

With determination, total count of the viable bacteria in the compound bacteria is 20×10⁸-30×10⁸. Wherein, there were 7% of *Rhodopseudomonas palustris*, 11% of *Bacillus subtilis,* 34% of *Saccharomyces cerevisiae*, 43% of *Lactobacillus acidophilus* and 5% of *Streptomyces jingyangensis.*

### Example 4 Preparative method of the complex microbial preparation

First level complex fermentation:
(1) 600ml of eggplant bottle strains of *Rhodopseudomonas palustris* was inoculated in 60L of the first level culture media for *Rhodopseudomonas palustris*, cultured at 36°C with a pH of 10 and light intensity of 4000Lux for 7 days to obtain first level bacteria of *Rhodopseudomonas palustris;*
   600ml of eggplant bottle strains of *Bacillus subtilis* was inoculated in 60L of the first level culture media for *Bacillus subtilis,* cultured at 34°C with a pH of 8 for 2 days to obtain first level bacteria *of Bacillus subtilis;*
   600ml of eggplant bottle strains of *Saccharomyces cerevisiae* was inoculated in 60L of the first level culture media for *Saccharomyces cerevisiae*, cultured at 30°C with a pH of 7 for 1 days to obtain first level bacteria of *Saccharomyces cerevisiae*;
   600ml of eggplant bottle strains of *Lactobacillus acidophilus* was inoculated in 60L of the first level culture media for *Lactobacillus acidophilus*, cultured at 34°C with a pH of 8 for 2 days to obtain first level bacteria of *Lactobacillus acidophilus;*
   600ml of eggplant bottle strains of *Streptomyces jingyangensis* was inoculated in 60L of the first level culture media for *Streptomyces jingyangensis*, cultured at 32°C with a pH of 8 for 5 days to obtain first level bacteria of *Streptomyces jingyangensis*;
(2) 60L of the first level bacteria of *Rhodopseudomonas palustris* was inoculated in 600L of second level culture media for *Rhodopseudomonas palustris*, cultured by intermittent aerobic cultivation at 36°C with a pH of 10 and light intensity of 4000Lux for 7 days to obtain fermenting bacterial suspension of *Rhodopseudomonas palustris*; with determination, viable count of the bacteria in the fermenting bacterial suspension is 5×10⁸-10×10⁸.
   60L of the first level bacteria of *Bacillus subtilis* was inoculated in 600L of second level culture media for *Bacillus subtilis,* cultured by aerobic cultivation at 34°C with a pH of 8 for 2 days to obtain fermenting bacterial suspension of *Bacillus subtilis;* with determination, viable count of the bacteria in the fermenting bacterial suspension is 30×10⁸-50×10⁸.
   60L of the first level bacteria of *Saccharomyces cerevisiae* was inoculated in 600L of second level culture media for *Saccharomyces cerevisiae*, cultured by aerobic cultivation at 30°C with a pH of 7 for 1 days to obtain fermenting bacterial suspension of *Saccharomyces cerevisiae*; with determination, viable count of the bacteria in the fermenting bacterial suspension is 5×10⁸-10×10⁸.
   60L of the first level bacteria of *Lactobacillus acidophilus* was inoculated in 600L of second level culture media for *Lactobacillus acidophilus*, cultured by static anaerobic cultivation at 34°C with a pH of 8 for 2 days to obtain fermenting bacterial suspension of *Lactobacillus acidophilus*; with determination, viable count of the bacteria in the fermenting bacterial suspension is 20×10⁸-30×10⁸.
   60L of the first level bacteria of *Streptomyces jingyangensis* was inoculated in 600L of second level culture media for *Streptomyces jingyangensis,* cultured by aerobic cultivation at 32°C with a pH of 8 for 5 days to obtain fermenting bacterial suspension of *Streptomyces jingyangensis*; with determination, viable count of the bacteria in the fermenting bacterial suspension is 30×10⁸-50×10⁸.
(3) Various components of comfermenting bacterial suspension were uniformly mixed in a 1000L fluid reservoir with a volume ratio of: 15% of *Rhodopseudomonas palustris,* 20% of *Bacillus subtilis,* 20% of *Saccharomyces cerevisiae,* 35% of *Lactobacillus acidophilus* and 10% of *Streptomyces jingyangensis,* obtaining 1000L of first level compound bacteria.

Second level complex fermentation:
3250 L of sterile water was added to 1000L of the first level compound bacteria to dilute it, then 200L of *Lactobacillus plantarum*, 200L of *Lactobacillus acidophilus*, 200L of *Saccharomyces cerevisiae* and 150L of brown sugar were added to mix uniformly in a fluid reservoir, fermented under sterile and closed condition, cultured at 35°C with a pH of 8 for 3 days to obtain 5000L of second level compound bacteria.

With determination, total count of the viable bacteria in the compound bacteria is 20×10⁸-30×10⁸. Wherein, there were 12% of *Rhodopseudomonas palustris,* 17% of *Bacillus subtilis,* 25% of *Saccharomyces cerevisiae,* 38% of *Lactobacillus acidophilus* and 8% of *Streptomyces jingyangensis.*

### Example 5 Preparative method of the complex microbial preparation

First level complex fermentation:
(1) 300ml of eggplant bottle strains of *Rhodopseudomonas palustris* was inoculated in 60L of the first level culture media for *Rhodopseudomonas palustris,* cultured at 28°C with a pH of 6 and light intensity of 3000Lux for 10 days to obtain first level bacteria of *Rhodopseudomonas palustris;*
   300ml of eggplant bottle strains of *Bacillus subtilis* was inoculated in 60L of the first level culture media for *Bacillus subtilis,* cultured at 28°C with a pH of 6 for 3 days to obtain first level bacteria of *Bacillus subtilis;*
   300ml of eggplant bottle strains of *Saccharomyces cerevisiae* was inoculated in 60L of the first level culture media for *Saccharomyces cerevisiae*, cultured at 25°C with a pH of 4 for 2 days to obtain first level bacteria of *Saccharomyces cerevisiae*;
   300ml of eggplant bottle strains of *Lactobacillus acidophilus* was inoculated in 60L of the first level culture media for *Lactobacillus acidophilus*, cultured at 28°C with a pH of 6 for 5 days to obtain first level bacteria *of Lactobacillus acidophilus*;
   300ml of eggplant bottle strains of *Streptomyces jingyangensis* was inoculated in 60L of the first level culture media for *Streptomyces jingyangensis*, cultured at 27°C with a pH of 6 for 7 days to obtain first level bacteria of *Streptomyces jingyangensis*;
(2) 60L of the first level bacteria *of Rhodopseudomonas palustris* was inoculated in 600L of second level culture media for *Rhodopseudomonas palustris*, cultured by intermittent aerobic cultivation at 28°C with a pH of 6 and light intensity of 3000Lux for 10 days to obtain fermenting bacterial suspension of *Rhodopseudomonas palustris*; with determination, viable count of the bacteria in the fermenting bacterial suspension is 5×10⁸-10×10⁸.
   60L of the first level bacteria of *Bacillus subtilis* was inoculated in 600L of second level culture media for *Bacillus subtilis,* cultured by aerobic cultivation at 28°C with a pH of 6 for 3 days to obtain fermenting bacterial suspension of *Bacillus subtilis;* with determination, viable count of the bacteria in the fermenting bacterial suspension is 30×10⁸-50×10⁸.
   60L of the first level bacteria of *Saccharomyces cerevisiae* was inoculated in 600L of second level culture media for *Saccharomyces cerevisiae*, cultured by aerobic cultivation at 25°C with a pH of 4 for 2 days to obtain fermenting bacterial suspension of *Saccharomyces cerevisiae*; with determination, viable count of the bacteria in the fermenting bacterial suspension is 5×10⁸-10×10⁸.
   60L of the first level bacteria of *Lactobacillus acidophilus* was inoculated in 600L of second level culture media for *Lactobacillus acidophilus*, cultured by static anaerobic cultivation at 28°C with a pH of 6 for 5 days to obtain fermenting bacterial suspension of *Lactobacillus acidophilus*; with determination, viable count of the bacteria in the fermenting bacterial suspension is 20×10⁸-30×10⁸.
   60L of the first level bacteria of *Streptomyces jingyangensis* was inoculated in 600L of second level culture media for *Streptomyces jingyangensis*, cultured by aerobic cultivation at 27°C with a pH of 6 for 7 days to obtain fermenting bacterial suspension of *Streptomyces jingyangensis*; with determination, viable count of the bacteria in the fermenting bacterial suspension is 30×10⁸-50×10⁸.
(3) Various components of comfermenting bacterial suspension were uniformly mixed in a 1000L fluid reservoir with a volume ratio of: 15% of *Rhodopseudomonas palustris,* 20% of *Bacillus subtilis,* 25% of *Saccharomyces cerevisiae,* 30% of *Lactobacillus acidophilus* and 10% of *Streptomyces jingyangensis,* obtaining 1000L of first level compound bacteria.

Second level complex fermentation:
2450 L of sterile water was added to 1000L of the first level compound bacteria to dilute it, then 150L of *Lactobacillus plantarum,* 150L of *Lactobacillus acidophilus,* 150L of *Saccharomyces cerevisiae* and 100L of brown sugar were added to mix uniformly in a fluid reservoir, fermented under sterile and closed condition, cultured at 30°C with a pH of 7 for 3 days to obtain 4000L of second level compound bacteria.

With determination, total count of the viable bacteria in the compound bacteria is 20×10⁸-30×10⁸. Wherein, there were 13% of *Rhodopseudomonas palustris,* 16% of *Bacillus subtilis,* 29% of *Saccharomyces cerevisiae,* 33% of *Lactobacillus acidophilus* and 9% of *Streptomyces jingyangensis.*

### Experimental example 1 the use of the complex microbial preparation according to the invention in producing medicaments for treatment of diabetes

1. General data: there are 103 out-patients, including 81 males and 22 females; age: 40-70 years old.
2. Diagnostic standards: according to the diagnostic standards established by WHO, most of the above patients were diagnosed as patients with mid-term or long-term diabetes.
3. Therapeutic method: the complex microbial preparation according to the invention produced in Example 1 was orally administered to patients with diabetes three times a day with 20 ml each time in 10 minutes after each meal. 15 days was taken as a course of treatment, 6 courses in total are required.

Notice: 1) shake up the medicine with the bottom up before taking it; 2) don't drink water in 1 hour after taking medicine; 3) don't need to go on a diet; 4) for a patient who are taking insulin, the dosage of the insulin can be reduced gradually in 7 days, and stop to take insulin after the blood glucose level returns to normal; 5) the dosage of other medicines for diabetes can be reduced gradually in 15 days, and stop to take the medicines after the blood glucose level returns to normal; 6 ) keep in dark place at room temperature.

### 4. Standards of curative effect evaluation

Significant effect: the level of fast blood glucose and blood glucose in 2 hours after meal are decreased to normal range; or the deceased level of fast blood glucose and blood glucose in 2 hours after meal is over 40% of the level before treatment; the level of glycosylated hemoglobin is decreased to normal range; or the deceased level of glycosylated hemoglobin is over 30% of the level before treatment.

Symptomatic relief: the deceased level of fast blood glucose and blood glucose in 2 hours after meal is over 20% of the level before treatment, but it does not reach the standards of significant effect; the deceased level of glycosylated hemoglobin is over 10% of the level before treatment, but it does not reach the standards of significant effect.

No effect: the level of fast blood glucose and blood glucose in 2 hours after meal are not decreased, or the decreased level does not reach the standards of symptomatic relief; the level of glycosylated hemoglobin is not decreased, or the decreased level does not reach the standards of symptomatic relief.

### 5. The results of treatment (see: Table 1)

**Table 1: Curative effect observation on patients treated with the complex microbial preparation according to the invention**

| Treatment duration | Significant effect | | Symptomatic relief | | No effect | | Effective rate, % |
|---|---|---|---|---|---|---|---|
| | Cases | Significant effect rate, % | Cases | Symptomatic relief rate, % | Cases | No effect rate, % | |
| 15 days | 21 | 20.39 | 59 | 57.28 | 23 | 22.33 | 77.67 |
| 45 days | 47 | 45.63 | 43 | 41.75 | 13 | 12.62 | 87.38 |
| 3 months | 61 | 59.22 | 36 | 34.95 | 6 | 5.83 | 94.17 |

### Typical cases:

Mr. Mao: male, 61 years old; suffering from diabetes for 18 years and taking insulin for 9 years; before taking the complex microbial preparation according to the invention, Mr. Mao went on a diet and was in poor health with 18.5 mmol/L of highest blood glucose level, 7.6 mmol/L of triglyceride and 9.4 mmol/L of total cholesterol. After taking the complex microbial preparation according to the invention for 15 days, Mr. Mao stopped to take insulin and the dosage of other medicines for diabetes were reduced by 50%; then Mr. Mao didn't go on a diet, and the level of blood glucose level, triglyceride and cholesterol were apparently decreased; with detection after 45 days, the health and diet for Mr. Mao were comprehensively returned to normal with 5.7 mmol/L of blood glucose, 2.3 mmol/L of triglyceride and 4.5 mmol/L of total cholesterol.

Mr. Liu: male, 57 years old; suffering from diabetes for 17 years and taking insulin for 6 years; before taking the complex microbial preparation according to the invention, Mr. Liu went on a diet and was in poor health with 16.9 mmol/L of highest blood glucose level, 6.9 mmol/L of triglyceride and 8.2 mmol/L of total cholesterol. After taking the complex microbial preparation according to the invention for 13 days, Mr. Liu stopped to take insulin and the dosage of other medicines for diabetes were reduced by 50%; then Mr. Liu didn't need to go on a diet, and the level of blood glucose level, triglyceride and cholesterol were apparently decreased; with detection after 45 days, the health and diet for Mr. Liu were comprehensively returned to normal with 4.6 mmol/L of blood glucose, 2.8 mmol/L of triglyceride and 5.3 mmol/L of total cholesterol.

### Industrial Applicability

The complex microbial preparation according to the invention can rapidly regulate metabolic function of human body, repair and recombine pancreatic islet cell mass, transform the redundant glucose in the blood, reduce the burden of impaired pancreatic β cells, gradually recover the function of the impaired pancreas islets, improve microcirculation perfusion , improve the velocity of nerve transmission so as to increase acral temperature, improve sleep of the patients with neurological complication, maintain steady state of the inter environment, keep the balance of organism, correct metabolic disorder and unbalance, can be used for treatment of diabetes and the complications and improving life quality of diabetic patients.

## Claims

1. Use of a complex microbial preparation, comprising 5-15% by volume of *Photosynthetic bacterium,* 10-20% by volume of *Bacillus,* 20-35% by volume of Yeasts, 30-45% by volume of *Lactobacillus* and 3-10% by volume of *Actinomycetes* in producing a medicament for treatment of diabetes.

2. The use according to claim 1, in which the complex microbial preparation comprises 5-10% by volume of *Photosynthetic bacterium,* 10-14% by volume of *Bacillus,* 30-35% by volume of Yeasts, 41-45% by volume of *Lactobacillus* and 3-8% by volume of *Actinomycetes*.

3. The use according to claim 2, in which the complex microbial preparation comprises 10% by volume of *Photosynthetic bacterium,* 10% by volume of *Bacillus,* 30% by volume of Yeasts, 45% by volume of *Lactobacillus* and 5% by volume of *Actinomycetes.*

4. The use according to any one of claims 1 to 3, wherein the *Photosynthetic bacterium* is *Rhodopseudomonas palustris*, the *Bacillus* is *Bacillus subtilis,* the Yeasts is *Saccharomyces cerevisiae*, the *Lactobacillus* is *Lactobacillus acidophilus* and the *Actinomycetes* is *Streptomyces jingyangensis.*

5. The use according to any one of claims 1 to 4, wherein the complex microbial preparation is obtained by a preparative method comprising the steps of first and second level complex fermentations of *Photosynthetic bacterium, Bacillus,* Yeasts, *Lactobacillus* and *Actinomycetes.*

6. The use according to claim 5, wherein the first level complex fermentation comprises steps of: (1) separately inoculating the *Photosynthetic bacterium, Bacillus,* Yeasts, *Lactobacillus* and *Actinomycetes* on first level culture media and conducting a first level cultivation to obtain first level bacteria of each component; (2) separately inoculating each of the obtained first level bacteria on second level culture media and conducting a second level cultivation to obtain fermenting bacterial liquids of each component; (3) mixing the five obtained fermenting bacterial liquids of each component to obtain first level compound bacteria.

7. The use according to claim 6, wherein in the step (2), the *Photosynthetic bacterium* are cultured by intermittent aerobic cultivation; the *Bacillus* are cultured by aerobic cultivation, the Yeasts are cultured by aerobic cultivation, the *Lactobacillus* are cultured by static anaerobic cultivation, and the *Actinomycetes* are cultured by aerobic cultivation.

8. The use according to claim 5, wherein the second level complex fermentation comprises steps of: adding sterile water to the first level compound bacteria to dilute it 2 to 4 times, then adding 10-20% of *Lactobacillus plantarum*, 10-20% of *Lactobacillus acidophilus*, 10-20% of *Saccharomyces cerevisiae* and 5-15% of brown sugar thereto, mixing uniformly in a fluid reservior, fermenting under sterile and closed condition, culturing with a pH of 5 to 8 at 25-35°C for 2 to 3 days to obtain second level compound bacteria.

9. The complex microbial preparation as defined in any one of claims 1 to 8 for use in the treatment of diabetes.

## Patentansprüche

1. Verwendung einer komplexen mikrobiellen Zubereitung, umfassend 5-15 Vol.-% eines *photosynthetischen Bakteriums,* 10-20 Vol.-% *Bacillus,* 20-35 Vol.-% Hefen, 30-45 Vol.-% *Lactobacillus* und 3-10 Vol.-% *Actinomycetes* zur Herstellung eines Medikaments zur Behandlung von Diabetes.

2. Verwendung nach Anspruch 1, wobei die komplexe mikrobielle Zubereitung 5-10 Vol.-% eines *photosynthetischen Bakteriums,* 10-14 Vol.-% *Bacillus,* 30-35 Vol.-% Hefen, 41-45 Vol.-% *Lactobacillus* und 3-8 Vol.-% *Actinomycetes* umfasst.

3. Verwendung nach Anspruch 2, wobei die komplexe mikrobielle Zusammensetzung 10 Vol.-% eines *photosynthetischen Bakteriums,* 10 Vol.-% *Bacillus,* 30 Vol.-% Hefen, 45 Vol.-% *Lactobacillus* und 5 Vol.-% *Actinomycetes* umfasst.

4. Verwendung nach einem der Ansprüche 1 bis 3, wobei es sich bei dem *photosynthetischen Bakterium* um *Rhodopseudomonas palustris,* bei *eacillus* um *Bacillus subtilis,* bei den Hefen um *Saccharomyces cerevisiae,* bei *Lactobacillus* um *Lactobacillus acidophilus* und bei *Actinomycetes* um *Streptomyces jingyangensis* handelt.

5. Verwendung nach einem der Ansprüche 1 bis 4, wobei man die komplexe mikrobielle Zubereitung durch ein Herstellungsverfahren erhält, welches die Schritte der ersten und zweiten Stufe komplexer Fermentierungen von einem *photosynthetischen Bakterium, Bacillus,* Hefen, *Lactobacillus* und *Actinomycetes* umfasst.

6. Verwendung nach Anspruch 5, wobei die erste Stufe der komplexen Fermentation die folgenden Schritte umfasst: (1) getrenntes Inokulieren von *photosynthetischem Bakterium, Bacillus,* Hefen, *Lactobacillus* und *Actinomycetes* auf einem Kulturmedium der ersten Stufe und Durchführen einer Kultivierung der ersten Stufe unter Erhalt einer ersten Stufe von Bakterien der jeweiligen Komponente, (2) getrenntes Inokulieren der jeweils erhaltenen ersten Stufen von Bakterien auf einem Kulturmedium der zweiten Stufe und Durchführen einer Kultivierung der zweiten Stufe unter Erhalt fermentierender bakterieller Flüssigkeiten der jeweiligen Komponente, (3) Mischen der fünf erhaltenen fermentierenden bakteriellen Flüssigkeiten der jeweiligen Komponente unter Erhalt von Verbindungsbakterien der ersten Stufe.

7. Verwendung nach Anspruch 6, wobei in Schritt (2) das *photosynthetische Bakterium* durch intermittierende aerobe Kultivierung kultiviert wird, *Bacillus* durch aerobe Kultivierung kultiviert wird, die Hefen durch aerobe Kultivierung kultiviert werden, *Lactobacillus* durch statische anaerobe Kultivierung kultiviert wird und *Actinomycetes* durch aerobe Kultivierung kultiviert wird.

8. Verwendung nach Anspruch 5, wobei die komplexe Fermentierung der zweiten Stufe die folgenden Schritte umfasst: Zugabe von sterilem Wasser zu den Verbindungs-bakterien der ersten Stufe zur 2- bis 4-fachen Verdünnung, dann Zugabe von 10-20% *Lactobacillus plantarum,* 10-20% *Lactobacillus acidophilus,* 10-20% *Saccharomyces cerevisiae* und 5-15% braunem Zucker dazu, homogenes Mischen in einem Flüssigkeitsreservoir, Fermentieren unter sterilen und geschlossenen Bedingungen und 2-bis 3-tägiges Kultivieren bei einem pH-Wert von 5 bis 8 und bei 25-35°C unter Erhalt von Verbindungsbakterien der zweiten Stufe.

9. Komplexe mikrobielle Zubereitung nach einem der Ansprüche 1 bis 8 zur Verwendung bei der Behandlung von Diabetes.

## Revendications

1. Utilisation d'une préparation microbienne complexe, comprenant 5-15% en volume de *Photosynthetic bacterium,* 10-20% en volume de *Bacillus,* 20-35% en volume de levures, 30-45% en volume de *Lactobacillus* et 3-10% en volume *d'Actinomycetes* pour la production d'un médicament pour le traitement du diabète.

2. Utilisation selon la revendication 1, dans laquelle la préparation microbienne complexe comprend 5-10% en volume de *Photosynthetic bacterium,* 10-14% en volume de *Bacillus,* 30-35% en volume de levures, 41-45% en volume de *Lactobacillus* et 3-8% en volume *d'Actinomycetes.*

3. Utilisation selon la revendication 2, dans laquelle la préparation microbienne complexe comprend 10% en volume de *Photosynthetic bacterium,* 10% en volume de *Bacillus,* 30% en volume de levures, 45% en volume de *Lactobacillus* et 5% en volume *d'Actinomycetes.*

4. Utilisation selon l'une quelconque des revendications 1 à 3, dans laquelle le *Photosynthetic bacterium* est *Rhodopseudomonas palustris,* the *Bacillus* is *Bacillus subtilis,* la levure est *Saccharomyces cerevisiae,* le *Lactobacillus* est *Lactobacillus acidophilus* et *l'Actinomycetes* est *Streptomycetes jingyangensis.*

5. Utilisation selon l'une quelconque des revendications 1 à 4, dans laquelle la préparation microbienne complexe est obtenue par une méthode de préparation comprenant les étapes de premier et second niveaux de fermentations complexes de *Photosynthetic bacterium, Bacillus,* levures, *Lactobacillus* et *Actinomycetes.*

6. Utilisation selon la revendication 5, dans laquelle le premier niveau de fermentation complexe comprend les étapes de : (1) inoculer séparément les *Photosynthetic bacterium, Bacillus,* Levure, *Lactobacillus* et *Actinomycetes* dans un milieu de culture de premier niveau et réaliser une culture de premier niveau pour obtenir une bactérie de premier niveau de chaque composant ; (2) inoculer séparément chacune des bactéries de premier niveau obtenues sur des milieux de culture de second niveau et mener une culture de second niveau pour obtenir des liquides de fermentation bactériens de chaque composant ; (3) mélanger les cinq liquides de fermentation bactériens de chaque composant pour obtenir un composé bactérien de premier niveau.

7. Utilisation selon la revendication 6, dans laquelle, dans l'étape (2), les *Photosynthetic bacterium* sont cultivés par culture aérobie intermittente ; les *Bacillus* sont cultivés par culture aérobie, les levures sont cultivées par culture aérobie, les *Lactobacillus* sont cultivés par culture anaérobie statique, et les *Actinomycetes* sont cultivés par culture aérobie.

8. Utilisation selon la revendication 5, dans laquelle le second niveau de fermentation complexe comprend les étapes de : ajout d'eau stérile au composé bactérien de premier niveau pour le diluer de 2 à 4 fois, puis ajouter 10-20% de *Lactobacillus plantarum,* 10-20% de *Lactobacillus acidophilus,* 10-20% de *Saccharomyces cerevisiae* et 5-15% de sucre brun à ceux-ci, mélanger uniformément dans un réservoir de fluides, fermenter sous condition stérile et fermée, cultiver à pH de 5 à 8 à une température de 25-35°C pendant 2 à 3 jours pour obtenir un composé bactérien de second niveau.

9. Préparation microbienne complexe telle que définie selon l'une quelconque des revendications 1 à 8, pour son utilisation dans le traitement du diabète.
